Europäisches Patentamt

European Patent Office

Office européen des brevets

(11) Publication number: **0 151 844**

**B1**

(12) **EUROPEAN PATENT SPECIFICATION**

(45) Date of publication of patent specification: **24.05.89**

(51) Int. Cl.⁴: **C 12 P 21/02, C 12 N 5/02**

(21) Application number: **84300857.4**

(22) Date of filing: **10.02.84**

(54) Process for in vitro production of serum-free and mitogen-free interleukin-2.

(43) Date of publication of application:
**21.08.85 Bulletin 85/34**

(45) Publication of the grant of the patent:
**24.05.89 Bulletin 89/21**

(84) Designated Contracting States:
**AT BE CH DE FR GB IT LI LU NL SE**

(56) References cited:
**EP-A-0 049 611**
**EP-A-0 077 571**

**CHEMICAL ABSTRACTS, vol. 98, no. 15, 11th April 1983, page 474, no. 123990d, Columbus, Ohio, US; J.L. PAULY et al.: "Studies of cultured human T lymphocytes. IV. Production of serum- and mitogen-free supernatants having high levels of the human T cell growth-promoter interleikin-2"**

(73) Proprietor: **HOOPER TRADING CO. N.V.**
**Handelscade 8**
**Curacao (NL)**

(72) Inventor: **Fabricius, Hans-Ake**
**P.O. 1409 Germanstrasse 17**
**D-7730 Villingen (DE)**
Inventor: **Stahn, Roland**
**Lankrechtstrasse 20**
**78 Freiburg (DE)**

(74) Representative: **Dixon, Donald Cossar et al**
**Gee & Co. Chancery House Chancery Lane**
**London WC2A 1QU (GB)**

**Description**

This invention relates to an improved method for producing T-cell growth factor (TCGF), also known as interleukin-2 (IL-2), in an *in vitro* culture system. More particularly, this invention relates to improvements in the *in vitro* culture production method for producing serum-free and mitogen-free interleukin-2 preparations disclosed in prior European Application No. 81304560.6, the disclosure of which is incorporated herein, by reference thereto; specifically this invention relates to the improvement wherein the cultivation is carried out under high oxygen concentration.

There has been an extensive amount of research in recent years regarding the immunopotentiating effects of various lymphokines, including interleukin-2. In July 1980, an International Workshop on Interleukin-2 was held in Geisenheim, Federal Republic of Germany, and a symposium publication based on this meeting was published in Behring Institute Mitteilunger, No. 67, December 1980.

Murine qnd human interleukin-2 have been partially purified and characterized as described, for instance, by S. A. Rosenberg, et al "In Vitro Growth of Murine T Cells III. Method for Separation of T Cell Growth Factor (TCGF) from Concanavalin A and Biological Activity of the resulting TCGF", J. of Immunological Methods, 33 (1980), pp. 339—350; J. Watson, et al "T-Cell Growth Factors: Interleukin-2", Immunology Today, December 1980, pp. 113—116; D. Mochizuki, et al, "Biochemical and Biological Characterization of Lymphocyte Regulatory Molecules. IV. Purification of Interleukin-2 from a Murine T Cell Lymphoma", J. of Immunology, Vol. 125, No. 6, (Dec. 1980), pp. 2579—2583; J. W. Mier and R. C. Gallo "Purification and some Characteristics of Human T-Cell Growth Factor (TCGF) from PHA-Stimulated Lymphocyte Conditioned Media".

The immunopotentiating effects of lymphokines, especially interleukin-2 have been described by several researchers, including, for example, Papermaster, et al "Preliminary Observations on Tumor Regressions induced by Local Administration of a Lymphoid Cell Culture Supernatant Fraction in Patients with Cutaneous Metastatic Lesions", J. of Immunology and Immunopathology, Vol. 5, pp. 31—47 (1976); Henney, et al "Interleukin-2 Augments Natural Killer Cell Activity", Nature Vol. 291, May 28, 1981, pp. 335—338; Lotze et al., "The In Vivo Distribution of Autologous Human and Murine Lymphoid Cells grown in T-Cell Growth Factor (TCGF); Implications for the Adoptive Immunotherapy of Tumors", J. of Immunology, Vol. 12, No. 4 (October 1980). The inventors own clinical studies have demonstrated the lack of any harmful side effects by the *in vivo* administration of interleukin-2 in tumor patients.

The importance of serum-free tissue culture systems has been recognized by several authors, for example, see B. W. Needleman and J. M. Weiler, "Human Lymphocyte Transformation induced by Mitogens and Antigens in a Serum-Free Tissue Culture System". J. of Immunological Methods, 44 (1981), pp. 3—14; H. S. Warren and R. G. Pembrey, "A Method for the Production and Quantitative Assay of Human Lumphokine Preparations", J. of Immunological methods, 41 (1981), pp. 9—21.

Production of a lectin-free murine interleukin-2 preparation is described by‘P. J. Spiess and A. A. Rosenberg, "A Simplified Method for the Production of Murine T-Cell Growth Factor Free of Lectin", J. of Immunological Methods, 42 (1981), pp. 13—222.

However, to date, it has not been possible to produce interleukin-2 on a large scale in culture systems.

Attempts to optimize the yield of Interleukin-2 *in vitro* cell cultures systems has largely centered on, for example, optimization of type of and mitrogen concentration, cell number and cell type, and other similar factors. See, for example, Jose M. Alvarez, et al "Human T Cell Growth Factor I. Optimal Conditions for its Production", J. of Immunology, Vol. 123, No. 3 (Sept. 1979), pp. 977—983. While some degree of success has been achieved by these methods, still greater yields would be highly beneficial to the study and applications of interleukin-2, particularly for long term and commercial use.

In conventional culture systems, e.g. flat dish, culture tube, or culture bottle, the cells being cultured tend to accumulate quickly at the bottom of the respective culture vessel. Cell accumulation in the *in vitro* production of Interleukin-2 (IL-2) appears to result during the mitogen (e.g. PHA) stimulation step when the cells tend to become "sticky" and clump together. This cell accumulation results in:

(1) rapid nutritional depletion of the lower layer of the culture medium;

(2) accumulation of metabolic products and synthesized proteins (some of which may be toxic or suppressive in respect to IL-2 production);

(3) intensive cell-to-cell contact, again possibly resulting suppression of IL-2 production by specific suppressor cells.

Media conditioned in such "conventional" systems, i.e. where cells are allowed to settle and where no circulation of the medium is provided, require concentration, of the order of 5 to 10-fold, to yield IL-2 levels high enough for the *in vitro* growth of cytotoxic IL-2 dependent T-cells. In fact, some batches require even higher concentration, while others show no activity at all.

It can, therefore, be appreciated that IL-2 production in these conventional culture systems is both generally low and highly unpredictable. This situation is highly undesirable where it is needed to produce IL-2 on a commercial scale.

Another economic drawback in the conventional culture systems as presently practiced for production of IL-2 from human sources is the high cost of obtaining the peripheral mononuclear blood cells and the resulting waste of the non-used portions of the starting whole blood supply.

It has been found by the present inventors that in the large scale production of IL-2 by *in vitro* culture systems, in which the medium in the culture vessels form layers, often in excess of several inches, a substantial barrier to oxygen diffusion (from the air of the air/$CO_2$ mixture in the incubator) into the liquid media is presented. In fact, liquid layers in excess of 3 to 5 millimeters show some significant barrier to oxygen penetration throughout the thickness of the liquid conditioning media.

These and other drawbacks are now avoided by the various embodiments of the present invention which are all improvements over the static *in vitro* cell culture system described in our copending Application No. 81304560.6.

Accordingly, in one aspect, the present invention provides a process in which the peripheral mononuclear blood cells (PBL) are packed on a porous support (e.g. nylon wool, Sepharose® gel, hollow fiber systems, etc.) and the liquid cell culture medium and atmosphere are recirculated throughout the incubation and replenished with fresh culture medium by diafiltration. The recirculation of only the culture fluids tends to prevent depletion of the nutrients in the lower layer of the culture medium and removes metabolic and synthesized products from the cell environment.

According to the present invention, especially useful for large scale production, in both static (e.g. flat dish) and dynamic (i.e. roller bottle) culture systems, the yield of IL-2 is improved 5 to 10-fold or more by carrying out the incubation steps under high oxygen concentrations, in particular with gaseous mixtures containing from about 70 to 95% oxygen, 5 to 15% carbon dioxide, and the remainder primarily nitrogen. In static culture systems the long diffusion distances for oxygen in the relatively deep liquid cell culture medium is compensated for by the higher oxygen concentration resulting in increased concentrations of IL-2 which are typically 5 times higher than the yields obtained in a conventional air/$CO_2$ gas mixture.

In the roller culture system, the presence of high oxygen concentrations provide reliably high yields and oxygen concentrations of IL-2 in the conditioned media, allowing the conditioned media to be diluted up to 50 times, preferably up to 5 to 10 times, for the *in vitro* growth of cytotoxic T-cells. In comparison, IL-2 conditioned media produced in roller culture with a normal air/$CO_2$ atmosphere have to be concentrated 0 to 5-fold to support growth of IL-2 dependent cytotoxic T-cells. This is, of course, still a significant improvement over the static culture systems.

Even in the embodiment using a porous support with packed cells instead of the roller culture, the higher oxygen concentration provides increased yields of IL-2 and offers the advantage of a more easily controlled oxygen supply by continuously oxygenating and otherwise reconditioning (e.g. pH, nutrients, product removal, etc.) the cell-free circulating culture medium.

In still another aspect of the invention, applicable to each of the above described embodiments, the economics of the process are substantially improved by using buffy coat cells for the peripheral mononuclear blood cells. Buffy coat cells have the advantage that they are obtained as a by-product from whole blood which has been treated to recover whole blood plasma and erythrocytes (red blood cells), and do not require addition of heparin to prevent blood clotting. Thus, the buffy coat cells which are normally discarded can be recovered at substantially no additional cost over the normal production of red blood cells and whole blood plasma from blood blanks. Moreover, the recovery of leukocytes is higher from buffy-coat cells than from PBL obtained by the procedures described in our prior applications and which are well known in the art.

The above and other objects of the invention, for achieving improved yields of serum-free and mitogen-free IL-2 are obtained by: (1) stimulating IL-2 producing primary cells which have been washed to remove substantially all serum proteins bound to the external cell membrane surface by incubating the cells in a liquid tissue culture medium supplement with serum protein and mitogen, under an oxygen-rich atmosphere comprising from about 70 to about 95% oxygen and from about 5 to 15% carbon monoxide (the remainder being primarily nitrogen with minor amounts of other inert gases normally found in air); (2) separating and washing the stimulated cells to remove substantially all of the serum protein and mitogen; and (3) conditioning the cells obtained in step (2) by incubating the cells in the presence of a serum protein-free and mitogen-free liquid tissue culture medium under an oxygen rich atmosphere as described in step (1).

This embodiment, using an oxygen rich atmosphere during the incubation (stimulation and conditioning) steps, can be applied to the roller culture system, or to a static, e.g. flat dish, tube, or non-rotating bottle, culture system. Recirculation of both the liquid tissue culture medium and the conditioned cells is also applicable to this embodiment.

In a preferred mode of carrying out the above described embodiment of the invention, the IL-2 producing cells are buffy-coat cells obtained as a by-product during the separation of red blood cells and plasma from whole blood by conventional techniques. According to this embodiment, the buffy-coat cells should be substantially fresh, e.g. no more than 2 to 3 hours old, when used in the production of IL-2.

The invention will now be described, in greater detail for a better understanding thereof, with the aid of the following non-limiting Examples.

The roller cultivation apparatus can be any conventional device for rolling bottles containing tissue culture medium and cells. Such apparatus is widely used in the field of biology, especially in virology for the production of viruses.

The cultivation process may be essentially the same in all other aspects as in the process described in our prior Application e.g. No. 81304560.6. Thus, the IL-2 producing cells may be any normal (i.e. non-cancer or non-tumor) primary cell system such as peripheral mononuclear blood lymphocytes which are obtained and washed as previously described. The cell donor may be sheep, pig, cattle or human. The temperature and atmosphere may also be those previously and conventionally used, e.g. 37°C and an air/5—15% $CO_2$ atmosphere.

However, for best results in terms of economy and yields of IL-2, particularly in applications for human use, the IL-2 producing cells can be buffy coat cells and the atmosphere should be oxygen enriched, for example 70—95% $O_2$, preferably 80—95% $O_2$ (percent by volume), with about 5 to 15%, preferably 5 to 10% carbon dioxide ($CO_2$) as a buffer, and the remainder is substantially nitrogen, with a minor amount of inert gases, such as argon, normally found in air.

Example 1

As a specific example of the culture process, peripheral mononuclear blood cells are first obtained as previously described. Briefly, human blood obtained from a blood bank is mixed with heparin, a clotting inhibitor. The blood is layered into Ficoll-hypaque® mixture-containing sterile tubes which are then centrifuged. The resulting layer of peripheral mononuclear blood cells formed above the Ficoll-hypaque® layer in each tube is drawn off carefully with a pipet. The cells are then pooled in a clean tube and diluted with, for example, RPMI 1640. The tube is centrifuged at speeds which do not rupture the cells, and the supernatant is discarded. The cells are then washed repeatedly with fresh portions of RPMI solutions in the same manner. The resulting washed cell pellet containing about 500—1000 million cells is diluted with about 5 to 10 ml RPMI 1640 solution.

The cells may then be seeded out at a density of about $3\times10^6$/ml in a tissue culture medium, e.g. RPMI 1640 containing about 4 micrograms of phytohemagglutinin (PHA) supplemented with 10—15% blood serum. In place of the blood serum, the plasmatic human IL-2 inducing protein (PHILIP) described in the copending European Application No. 83304302.9 the disclosure of which is incorporated herein by reference thereto, may be used.

At this point, the cell and lectin containing culture medium is transferred to roller culture bottles. Bottles ranging in diameter of from 4 to 5 inches (10.16—12.70 cm) are used, and each bottle is filled with from 50 to 200 ml of culture medium. The roller bottles, after being filled with an atmosphere containing 90% $O_2$, 5% $CO_2$ and 5% $N_2$, are placed in an incubator equipped with the roller apparatus. The temperature in the incubator is maintained at about 37°C. The rollers are rotated at about 25 r.p.m. (a roller bottle speed of about 6 r.p.m.), and the cells are incubated for about 4 to 8 hours, preferably about 4 hours.

Thereafter, the bottles are removed from the incubator, the stimulated cells containing culture medium decanted into centrifuge tubes, and the cells sedimented and washed three times in the conventional manner as previously described, and then seeded out into fresh culture medium, again at a cell density of about 3,000,000/ml. In this case, the cell containing culture medium, which is not supplemented with PHA or serum proteins, is again transferred into the roller bottles in 50 to 200 ml portions and incubated under the same conditions used in the stimulation step, except that the time of the conditioning incubation is increased to about 18 to 24 hours, preferably about 20 hours.

Thereafter, the medium is again transferred from the roller bottles to centrifuge tubes and processed as in our previous Application (see Example 1 of No. 81304560.6). The cells can be reutilized in subsequent PHA restimulation and reconditioning for at least 5 additional cycles.

The IL-2 activity of the conditioned culture supernatants from the roller bottle plus 90% $O_2$, and from conventional PHA stimulation in 50 ml Falcon blue cap plastic tubes with normal atmospheric oxygen (air)+10% $CO_2$ (Comparative Example 1), were tested (three replications each) and the results are shown in the following table:

IL-2 Activity in conditioned culture supernatants

|  | Preparation procedure | |
|---|---|---|
|  | Plastic tubes +air/$CO_2$ (Comp. Ex. 1) | Roller bottles +90% $O_2$ (Ex. 1) |
| Cells per well×$10^{-3}$ | 95±12* | 243±27* |
| (dilution factor) | (1:4) | (1:8) |

*Average of 3 experiments.

Comparative Examples 2—3

Example 1 is repeated, except that the rollers were rotated at speeds of only about 15 r.p.m. The results are substantially the same as in Comparative Example 1, and substantial cell clumping is observed.

Example 2

Example 1 is repeated, except that buffy coat cells are used in place of the PBL cells. The buffy coat cells are obtained as follows: blood is collected in a vessel containing a preservative for the erythrocytes, e.g. ACD (Adenine, citrate, dextrose) which is a standard solution for preserving erythrocytes. The use of heparin, which is needed for the IL-2 production in whole blood conserves, is not necessary. After collection, the fresh blood specimen is centrifuged at 1000 to 1500×G for 20 minutes. The erythrocytes will sediment and the leukocytes will form a thin layer (buffy-coat) on the erythrocyte sediment. Thereafter, the buffy-coat is aspirated off the erythrocyte layer and used for the production of IL-2. The leuko- and erythrocyte-free plasma can be collected and used, for example, for industrial purposes or for the infusion as whole plasma into patients. The sedimented erythrocytes can be used for clinical purposes (e.g. blood transfusion), as usual. Most blood blanks prepare erythrocyte-concentrates in the way described above, only with the difference that the buffy-coat is not collected. The leukocytes are generally considered worthless. The blood conserve usually stands in a refrigerator for some days or weeks before it is used. During this time, the leukocytes will die. Using these buffy-coat cells, it is possible to obtain raw material for the IL-2 production, almost without additional costs for the blood bank.

When the leukocytes are now processed for IL-2 production, they are separated from contaminating erythrocytes and thrombocytes by a Ficoll-hypaque® gradient in exactly the same manner as previously described. The cell number recovered will usually be about 20% higher than the cell number recovered from a heparin-conserve. It is very important that the buffy-coat cells are absolutely fresh, when they are stimulated for the IL-2 production. They should not be older than 2 to 3 hours. It has been found, quite unexpectedly, that the use of buffy-coat has one limitation: if one tries to produce IL-2 in buffy-coats under normal oxygen concentration, no sufficient production will be found. One prerequisite, therefore, is the use of an oxygen rich atmosphere in the same manner as previously described. The result obtained is substantially the same as in Example 1.

Comparative Example 4

When Example 1 is repeated, except that buffy-coat cells are used in place of the PBL cells, substantially no IL-2 production is observed. This Example shows that buffy coat cells require high oxygen concentrations during incubation, although the reason for this has not been fully elucidated.

The IL-2 containing supernatants obtained by the improved procedures of this invention, after filtering, e.g. on an Amicon YM-10® filter as described in our prior Applications, is a highly purified product. Product purity of the IL-2 containing conditioned supernatant, can be demonstrated by the presence of less than twenty protein bands when the supernatant is subjected to gel electrophoresis or similar analytical evaluation. The protein content of the filtered supernatants usually ranges from about 10 to 100 µg/ml, due to the inherent differences in IL-2 producing capacity from cell batch to cell batch. That is, the cells of each donor have inherently different capacities to produce IL-2 by mitogen stimulation, although the reasons for these differences have not been established.

Accordingly, it is recognized that the present invention provides significant improvements in yield and in overall economy of the inventor's original procedure for the in vitro production of high purity serum-free and mitogen (e.g. lecitin-) free IL-2 containing conditioned supernatants. These supernatants are useful in the treatment of patients having depressed natural killer cell function, in the diagnosis and treatment of immune deficiency in tumor patients, and in general for any of the utilities previously disclosed in Application No. 81304560.6 or by other authors.

Claims

1. A process for producing a serum-free and mitogen - free interleukin - 2 containing supernatant, comprising the steps of (1) stimulating interleukin-2 producing cells by incubating the cells in a liquid tissue culture medium supplemented with serum protein and mitogen; (2) separating and washing the stimulated cells to remove substantially all of the serum protein and mitogen; and (3) conditioning the washed cells by incubating the cells in the presence of fresh liquid tissue culture medium in the absence of serum protein and mitogen to thereby transfer the interleukin-2 into the liquid phase; characterised in that the incubation steps are carried out under an oxygen rich atmosphere comprising at least 70% oxygen and at least 5% carbon dioxide.

2. A process as claimed in Claim 1, wherein the oxygen rich atmosphere comprises 80 to 95% $O_2$, 5—10% $CO_2$ and the remainder is substantially nitrogen.

3. A process as claimed in Claim 1 or Claim 2, wherein the interleukin-2 producing cells are buffy-coat cells obtained from a human donor.

Patentansprüche

1. Verfahren zur Gewinnung eines serumfreien, mitogenfreien, Interleukin-2 enthaltenden Ueberstandes (Supernatant) mit den Schritten:
(1) Stimulieren von Interleukin-2 erzeugenden Zellen durch Inkubieren der Zellen in einem flüssigen Gewebekulturmedium, das mit Serumprotein und Mitogen ergänzt ist;
(2) Abtrennen und Waschen der stimulierten Zellen zur Entfernung praktisch des gesamten Serumproteins und Mitogens; und
(3) Konditionieren der gewaschenen Zellen durch Inkubieren der Zellen in Gegenwart von frischem flüssigem Gewebekulturmedium ohne Serumprotein und Mitogen, um dadurch das Interleukin-2 in die flüssige Phase zu überführen;
dadurch gekennzeichnet, dass die Inkubierschritte in einer sauerstoffreichen Atmosphäre durchgeführt werden, die mindestens 70% Sauerstoff und mindestens 5% Kohlendioxid enthält.

2. Verfahren nach Anspruch 1, bei welchem die sauerstoffreiche Atmosphäre 80 bis 95% $O_2$, 5—10% $CO_2$ und als restlichen Anteil im wesentlichen Stickstoff enthält.

3. Verfahren nach Anspruch 1 oder Anspruch 2, bei welchem die Interleukin-2 erzeugenden Zellen von einem Humandonor gewonnene Buffycoat-Zellen sind.

**Revendications**

1. Un procédé pour la production d'une phase surnageante contenant de l'interleucine-2 exempte de sérume et de mitogène, comprenant les étapes de 1) stimuler les cellules de production de l'interleucine-2 par incubation des cellules dans un milieu de culture de tissus liquide auquel a été ajouté du sérum protéine et du mitogène; 2) séparer et laver les cellules stimulées pour éliminer substantiellement tout le sérum protéine et le mitogène; et 3) conditionner les cellules lavées par incubation des celles en présence de milieu de culture de tissus liquide frais en absence de sérum protéine et de mitogène pour ainsi transférer l'interleucine-2 dans la phase liquide; caractérisé en ce que les étapes d'incubation sont effectuées dans une atmosphère riche en oxygène comprenant au moins 70% d'oxygène et au moins 5% de dioxyde de carbone.

2. Un procédé selon la revendication 1, dans lequel l'atmosphère riche en oxygène comprend 80 à 95% $O_2$, 5—10% $CO_2$ et le reste étant substantiellement de l'azote.

3. Un procédé selon la revendication 1 ou la revendication 2, dans lequel les celles de production de l'interleucine-2 sont des cellules "buffy-coat" obtenues d'un donneur humain.